# EUROPEAN PATENT SPECIFICATION

(11) **EP 1 211 977 B1**
(45) Date of publication and mention of the grant of the patent: **18.05.2011**
(21) Application number: 00948232.4
(22) Date of filing: 25.07.2000
(51) Int. Cl.: A61B 5/103

(54) **Apparatus for the detection of physiological distress in a patient**
Vorrichtung zur Erfassung physiologischer Störungen
Appareil de detection de la détresse physiologique

(30) Priority: 26.07.1999 IL 13110899; 04.08.1999 IL 13124599
(43) Date of publication of application: 12.06.2002
(73) Proprietor: Cardiosense Ltd., Nesher 36601 (IL)
(72) Inventor: SHANI, Haim, 73142 Shaham (IL); SHAVIT, Ittai, 22401 Nahariya (IL)
(74) Representative: Becker Kurig Straus
(86) International application number: PCT/IL2000/000443
(87) International publication number: WO 2001/006926

(56) References cited:
- US-A- 3 698 382
- US-A- 4 494 550

## Description

### Field of the Invention

The present invention relates to the field of medical diagnosis instrumentation. More particularly, the invention relates to an apparatus, for non-invasive detection and monitoring of physiological states, for the diagnosis of shock, pre-shock and cardio-pulmonary distress in a patient, and of recovery of a patient from shock pre-shock and cardio-pulmonary distress by monitoring changes in the capillary flow in skin areas of peripheral body organs.

### Background of the Invention

The normal color of the skin in most locations of the human body is between pink and red. This color is mainly determined by the amount of blood flowing in the capillaries, through which blood flows from the arterioles to the venules. The present invention is based on a non-invasive detection of hemodynamic changes in (and particularly the reduction of) the skin arteriolar-capillary flow during the states of shock, pre-shock and cardio-pulmonary distress.

From US 3 698 382 and US 4494550 there are known apparatus for measuring veno capillary filling time and blood flow disorders.

Document US-A-4 494 550 represents the most relevant prior art.

The physiological principle of the invention is shock: Shock is a clinical syndrome. The diagnosis of shock is based on clinical appreciation of the presence of inadequate blood flow (perfusion) to organs and tissues. In most cases of shock the condition is reversible, but if lasts to long, it can cause an irreversible damage to the tissues and in severe cases even death. One of the most common types of shock is hypovolemic shock, which is the result of volume loss. For instance, massive blood loss (hemorrhage) or loss of body fluids due to severe dehydration, vomiting or diarrhea. An early compensatory mechanism in hypovolemic shock is increasing the resistance of the skin tissue arterioles in order to reduce blood perfusion to the skin tissue enabling an adequate perfusion to vital organs (brain, heart, and kidneys). The process of increasing arteriolar resistance is called vasoconstriction and is done by constricting the arteriolar walls and diminishing the vessel's diameter. This phenomenon of peripheral arteriolar vasoconstriction appears in some other types of shock (such as in shock as a result of heart failure). The vasoconstriction of the skin tissue arterioles and the reduction if the arteriolar blood flow leads to a reduction of the capillaries blood flow (poor perfusion to the skin). An important clinical sign of poor perfusion to the skin is the estimation of Capillary Filling Time (CFT). Upon applying minor pressure on a specific area of the skin, the capillaries under the depressed area collapse and blood is forced therefrom (blanched), causing the skin color under the depressed area to become white. Upon releasing the pressure abruptly, blood flows back into the capillaries and the original skin color returns. The CFT is defined as the estimated time for the original skin color to return after blanching. In the clinical practice, prolongation of the CFT of more than 2 seconds is indicating a state of poor skin perfusion (shock) or a cold ambient temperature. If the condition of shock continues, the arteriolar vasoconstriction increases even further and the prolongation of the CFT continues. On the other hand, appropriate treatment of shock decreases the vasoconstriction and shortens the CFT. Similar homodynamic changes occur in some other types of shock.

Another type of shock is associated with cardiovascular failures. Cardiovascular failure results from a substantial damage to the heart muscle (such as during an acute myocardial infarct), to a major blood vessel (such as in rupture of the aorta), or to other component of the heart (e.g., valvular damage to the endocard).

According to this physiological principle of hemodynamic compensation in shock, an accurate measuring of the CFT is a direct non-invasive method to evaluate perfusion in the condition of shock. The other known non-invasive methods to evaluate shock are:
Blood pressure: an indirect parameter of shock (do not evaluate perfusion). However, the measurement of blood pressure identifies shock only in late stages (de-compensated shock).
-Heart rate: an indirect parameter of shock (do not evaluate perfusion). The specificity of this measurement is low because heart rate is also increases in other common physiological conditions (e.g. anxiety, pain). Therefore, a method that enables an early identification of shock, and the monitoring of the severity of the shock and the patient immediate response to treatment (shortening of CFT is a proof of an improvement in peripheral perfusion) is desired.
early hypovolemic shock, myocardial ischemia and Cardio-pulmonary distress are all associated with some reduction of arteriolar-capillary flow in skin areas. Early hypovolemic shock is in fact an early stage of shock, in which the blood pressure of the patient is usually normal. It is therefore desired to detect such early stage in order to provide an appropriate medical treatment at this stage, so as to prevent deterioration to shock state. Since peripheral arteriolar vasoconstriction is an early manifestation of hypovolemic shock, the detection of CFT prolongation is a diagnosis of an early shock state.

Another state, in which inadequate blood supply is provided to the heart muscle is myocardial ischemia. Peripheral arteriolar vasoconstriction is a known manifestation of myocardial ischemia. In many cases myocardial ischemia is the first stage in the evaluation of acute damage to the heart muscle and/or of cardiovascular failure. The clinical manifestations of this condition are various and sometimes misleading. It is therefore desired to detect such first stage in order to provide an appropriate medical treatment at this stage, so as to prevent deterioration to a irreversible damage to the heart muscle (i.e., to a myocardial infarct).

Cardiopulmonary distress refers to diseases or medical conditions that induce impairment in the cardiopulmonary function that causes peripheral arteriolar vasoconstriction, for example, congestive heart failure, chronic lung disease, drug effect or side effect, or drug toxicity.

Other physiological distress which is associated with the reduction of arteriolar-capillary flow in skin areas is dehydration, which may be severe as a result of vomiting, diarrhea, severe burns or extreme environmental conditions. Such physiological conditions involve increased blood viscosity resulting from significant loss of fluids, which reduces the arteriolar-capillary flow. In addition, medical or surgical conditions, which comprise a reduction in blood flow to a limb (e.g., peripheral vascular disease, reconstruction of skin flaps, etc.) is also associated with the reduction of arteriolar-capillary flow in skin areas and prolongation of the CFT.

The diagnosis in most of the aforementioned conditions is difficult because the clinical signs are minimal, or even absent. The biological response in these conditions is the constriction of the arteriolar walls, which results in the reduction of the skin capillary flow and prolongation of the CFT. This CFT prolongation is detected and monitored.

US Patent 3,698,382 discloses an apparatus for measuring the veno-CFT, which employs intermittent and uniform pressure to the skin of a patient. The apparatus comprises a light source for illuminating a skin area and photoelectric monitoring means that is sensitive to the coloration of the skin area. The apparatus measures the rate of return of color to the skin area from pressure release. However, this apparatus suffers from interference of external light and requires an opaque housing for the monitoring means. In addition, the mechanical arrangement required for maintaining uniform pressure, required for better accuracy is cumbersome. In addition, US Patent 3,698,382 does not detect any continuos changes of the CFT (i.e., does not provide ΔCFT/Δt- curve)

All the methods described above have not yet provided satisfactory solutions to the problem of providing an apparatus for the diagnosis of shock, pre-shock and cardio-pulmonary distress in a patient and of recovery of a patient from actual state of shock, which is accurate and simple to use.

It is an object of the present invention to provide a non-invasive apparatus for the diagnosis of pre-shock and cardio-pulmonary distress pre-shock state in a patient and of recovery of a patient from actual state of shock, pre-shock and cardio-pulmonary distress, which overcomes the drawbacks of prior art.

It is a further object of the present invention to provide a non-invasive apparatus for the diagnosis of pre-shock and cardio-pulmonary distress pre-shock state in a patient and of recovery of a patient from actual state of shock, that does not require a uniform pressure on the skin.

It is still another object of the present invention to provide a non-invasive apparatus for the diagnosis of pre-shock and cardio-pulmonary distress pre-shock state in a patient and of recovery of a patient from actual state of shock, that provides an indication to the user whenever the magnitude and/or the duration of the pressure applied on the skin area is not sufficient.

It is still another object of the present invention to provide a non-invasive apparatus for the diagnosis and early detection of cardio-pulmonary distress in a patient.

It is still another object of the present invention to provide a non-invasive apparatus for the diagnosis and early detection of cardiovascular failure in a patient.

It is still another object of the present invention to provide a non-invasive apparatus for the diagnosis and early detection of myocardial ischemia in a patient.

It is still another object of the present invention to provide a non-invasive apparatus for the diagnosis and early detection of dehydration state in a patient.

It is yet a further object of the present invention to provide a non-invasive apparatus for a sensitive identification of clinical deterioration or improvement of the patient and the effectiveness of the treatment.

Other objects and advantages of the invention will become apparent as the description proceeds.

### Summary of the Invention

The present invention is directed to an apparatus of claim 1,
- post surgical skin states.

Preferably, the apparatus further comprises means for illuminating area of the skin to be gauged for color, with light radiation in a level being sufficient for enabling the image acquisition means to discriminate between colors.

Preferably, the apparatus comprises circuitry for producing an electrical signal representative of the color of the area; circuitry for storing the value of the electrical signal which corresponds to the original color of the area; a processor for processing data collected by the transducer and/or by the image acquisition means in combination with the stored data, for measuring the filling time after releasing the pressure; and a display for graphically displaying the processed data. The apparatus further comprises a transducer for applying pressure on the area, and for obtaining a signal value which corresponds to maximum whitening of the area; circuitry for sampling the image acquisition means for acquiring an image of an area of the skin of the patient, to be gauged for color; circuitry for sampling the value of the electrical signal at a predetermined rate during the measurement and for storing the sampled values; circuitry for providing an alert signal whenever the strength and/or the duration of the applied pressure is not sufficient for obtaining maximum whitening; and a device for automatically applying and releasing pressure.

Preferably, the apparatus further comprises a rigid transducer containing the image acquisition means and having a transparent wall being in contact with a limb of the patient, for applying a controlled pressure on the surface of the limb.

### Brief Description of the Drawings

The above and other characteristics and advantages of the invention will be better understood through the following illustrative and non-limitative detailed description of preferred embodiments thereof, with reference to the appended drawings, wherein:
- Fig. 1A schematically illustrates the structure of a skin color sensing apparatus for the diagnosis of pre-shock state in a patient by measuring the capillary filling time and rate, according to a preferred embodiment of the invention;
- Fig. 1B schematically illustrates the structure of a skin color sensing device for the diagnosis of pre-shock state in a patient by measuring the capillary filling time and rate, according to a preferred embodiment of the invention;
- Fig. 2 is a block diagram of an apparatus for the diagnosis of pre-shock state in a patient by measuring capillary filling time and rate, according to a preferred embodiment of the invention;
- Fig. 3A is a graphical representation of the measurement results of the CFT, according to a preferred embodiment of the invention;
- Fig. 3B is a graphical representation of the CFT as a function of the level of shock, for obtaining inferences related to the trend of the patient's physiological condition in reaction to medical treatment, according to a preferred embodiment of the invention; and
- Fig. 4 schematically illustrates the use an apparatus for the diagnosis of pre-shock state in a patient, according to a preferred embodiment of the invention.

### Detailed Description of Preferred Embodiments

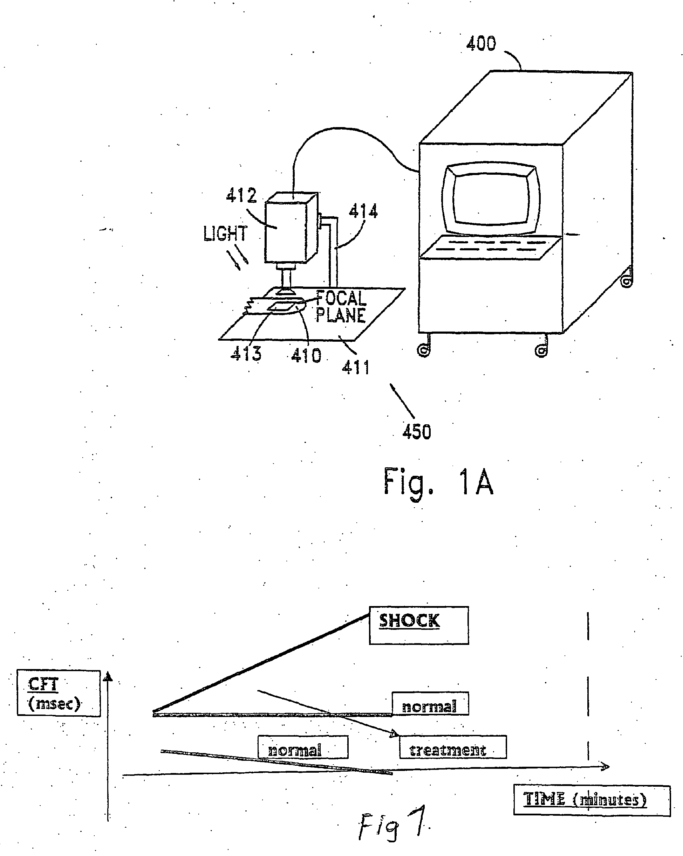

Fig. 1A schematically illustrates the structure of a skin color sensing apparatus for the diagnosis of pre-shock state in a patient by measuring the capillary filling time and rate, according to a preferred embodiment of the invention. The device 450 comprises a color video camera 412, which is fixed in place by an holder 414, above a rigid surface 411 (such as a table), to which the limb 410 of the patient is attached. The limb may be, for example, the patient's finger. The position of the camera is adjusted so that the skin area 413, which is photographed by the camera 412 for the purpose of the CFT measurement, is in, or close to, the focal plane of the lens. Pressure may applied on, and released from, the skin area 413 manually (e.g., by hand), or by using a mechanical apparatus, which may be automatically controlled.

Background light illuminates the skin area 413, and the light reflected from the surface of the skin area 413 is received in the lens of the camera 412. A minimal illumination level of 0.2 lux is sufficient, for most of the currently available modern cameras for color discrimination. The camera 412 produces an electrical signal having a magnitude, which corresponds to the color of the image, received by the camera. In the event that the illumination level of the background light is not sufficient, the skin area 413 can be illuminated with a light source, such as a conventional lamp or a Light Emitting Diode (LED).

A sensing device 100 is connected to a processing and display unit 400 with electrical cord, through which the CFT data is fed for processing and display. The processing and display unit 400, which may be a personal computer that uses a control and processing software, processes the data received in the lens of the camera 412, and calculates the CFT total time and rate. Pressure is applied and released manually by the user, according to indications and/or instructions provided by the processing and display unit 400. The processing and display unit 400 may further comprise control circuitry for controlling automated application of pressure. The control circuitry may also be used to select a specific area for processing, taken from the imaged skin area. Such selection may be carried out, for example by software, which controls the processing. The sensing device may also be attached to other locations in the patient's body that are rich in subcutaneous blood vessels, such as to the lip or to the ear lobe, for measuring the CFT.

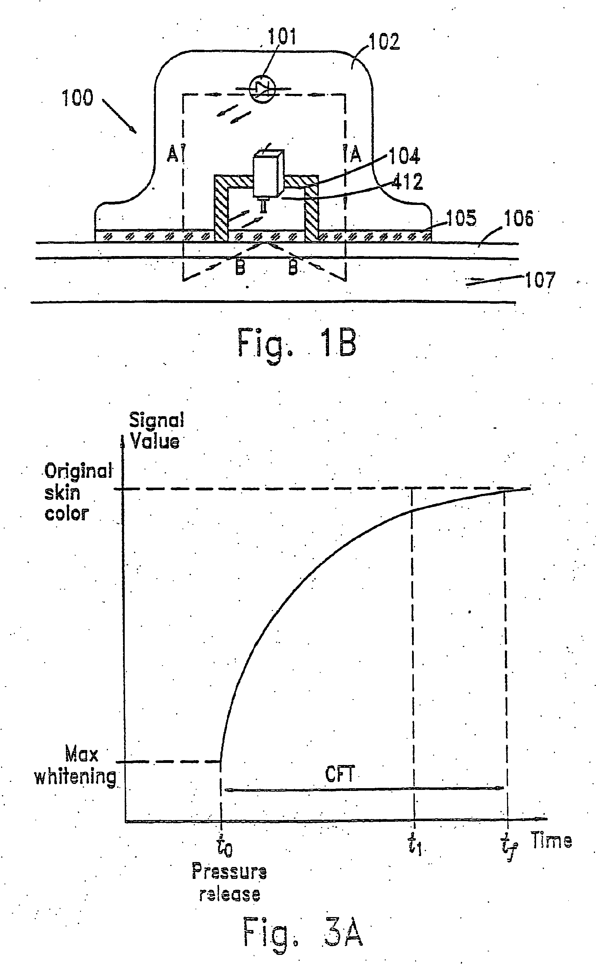

Fig. 1B schematically illustrates the structure of a skin color sensing device for the diagnosis of pre-shock state in a patient by measuring the capillary filling time and rate, according to a preferred embodiment of the invention. The device 100 comprises a color video camera 412, which is contained in a transparent external housing 102, so that most of the background light enters through the external housing and illuminates the skin surface 106.

The device 100 may further comprise an optional light source 101, such as a LED) that is operated by a power supply during measurement, when the background illumination level is not sufficient to enable the camera 412 to discriminate between colors. The external housing 102 may be light reflecting with an opening in its bottom side, so that most of the light radiation emitted from the light source 101 is directed toward the bottom side in one direction "A", wherein this embodiment is not claimed. The external housing 102 may also comprises an opaque internal housing 104, having an opening in its bottom side, so as to enable light radiation to enter into the opaque internal housing space only from its bottom side. Using this structure, the camera 412 is assembled in the internal housing 104 for receiving most of the light reflected from the skin. The bottom sides of the external housing 102 and the internal housing 104 are aligned and covered with a transparent rigid layer 105, which is used to apply pressure on the skin while enabling light to pass through in both directions.

The transparent rigid layer 105 is brought in contact with the exterior layer 106 of the skin. Pressure is applied manually or automatically on the external housing 102 toward the skin surface in a perpendicular direction. The external housing delivers the pressure to the transparent rigid layer 105, which transfers it farther, through the exterior layer 106, to the interior layer 107 of the skin, that contains most of the subcutaneous blood vessels (capillaries). As a result, if the magnitude of the applied pressure is sufficient and maintained for sufficient period of time, blood is forced out of the capillaries that are under pressure, and the color of the interior layer 107 of skin becomes much brighter (i.e. close to white). The background light and/or the light radiation emitted from light source 101 penetrates into skin, and partially reflected back in direction "B", into the internal housing 104. The degree of reflection from the interior layer 107 is inversely related to the blood flow in the capillaries under pressure, since blood absorbs light. The reflected light enters the lens of the camera 412, which produces an electric signal, the magnitude of which is responsive to the instantaneous color of the skin. The position of the camera 412 within the device 100 is determined so that the exterior surface of the transparent rigid layer 105 is essentially in the focal plane of the camera 412. This positioning results in a clear and focused image that is received in the lens of the camera. A focused image improves the distinction between colors, and therefore, improves the resolution and the accuracy of the measurement.

Under no pressure (i.e., full blood flow), the skin color is normally pink, and less light radiation is reflected back from the capillaries. Under pressure, (i.e., no blood flow), the skin color is normally white, and more light radiation is reflected back from the capillaries. Therefore, changes in the magnitude of the electric signal produced by the camera 412, provides an accurate measure of the capillary filling time and rate upon releasing the pressure from the skin. The device 100 is connected to a power supply for operating the optional light source 101 and to data collection, processing and display circuitry for processing the signals provided by the camera 412 and displaying the measurement results.

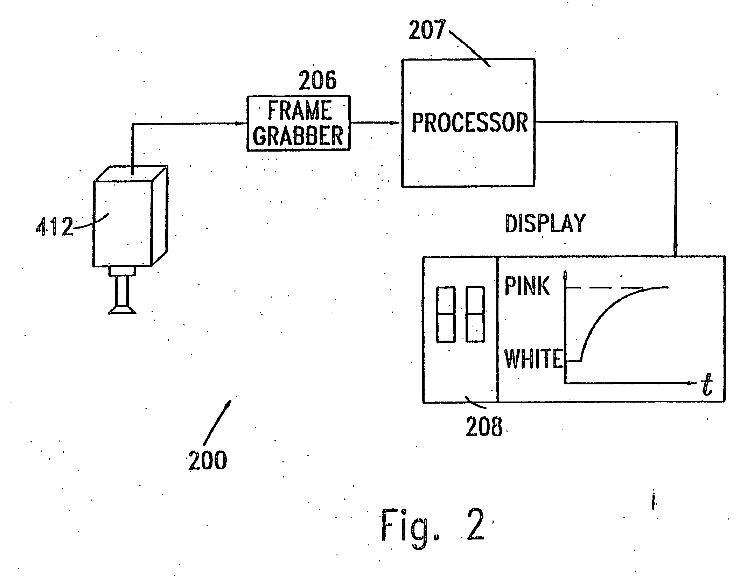

Fig. 2 is a block diagram of an apparatus for the diagnosis of pre-shock state in a patient by measuring capillary filling time and rate, according to a preferred embodiment of the invention. The apparatus 200 comprises the color video camera 412, and a frame grabber 206, for capturing the image received by the camera 412. The light reflected from the skin surface, is converted by the camera 412 to a corresponding video signal, such as a Composite Video or an Red-Green-Blue (RGB) Video signal (according to the type of camera that is used), that represents the received image. The video signal is fed into an electronic circuit (e.g., a Frame Grabber or a Video Capture circuit) which decodes the video signal and converts it into a corresponding array of digital values. The array is stored in a memory. Each memory cell, containing a digital value, represents the light intensity and the color of a portion of the received image. The camera 412 updates the image at a rate of 50 times per second, and therefore, the image information, generated by the frame grabber 206 and stored in the memory array, is also updated at the same rate. A rate of 50 times per second usually corresponds to video cameras, which are compatible with Pulse Alteration by Line (PAL) video encoding standard. A rate of 60 times per second usually corresponds to video cameras, which are compatible with National Television System Committee (NTSC) video standard. Faster color video cameras, which update the image in higher rate, can also be used.

The digital data is fed into a digital processor 207, which analyzes the data and display the results on a suitable display. The processor 207 samples a desired area of the image, which contains most of the tested skin area. At the next step, the processor 207 calculates the intensity of the essentially pink/red light, reflected by the tested skin area. The intensity of the reflected light is processed and normalized to a baseline, which may be the normal color of the patient's skin, when no pressure is applied. The image information is updated in a rate determined by the type of color video camera that is used. Therefore, the processor 207 continuously calculates the normalized intensity.

The display 208 comprises the display of the calculated results of the normalized intensity (i.e., the CFT), and of a graphical representation of the measurement process as a function of time. The graphical representation provides an indication whether the measurement results are reasonable or not, and if desired, the measurement can be repeated. According to a preferred embodiment of the invention, other data processed results, such as statistical data, can be also displayed to provide indications related to the reaction of the patient to medical treatment etc.

Fig. 3A is a graphical representation of the measurement results of the CFT, according to a preferred embodiment of the invention. At the first stage, no pressure is applied on the skin, and therefore the apparatus 200 can carry out calibration of the initial skin color of the patient. The value of the calibration is stored for use at the end of the measurement. The calibration process is essential, since the normal color of the skin is individual and different from patient to patient. At the second stage, pressure is applied at a magnitude and duration that are sufficient to obtain maximum whitening of the skin color in the depressed area. The processor can be programmed to provide a warning signal (such as a beep) to the user, that the pressure is not sufficient or shorter than required. Obtaining maximum whitening of all the depressed area can be an indication to sufficient pressure. Longer and/or stronger pressure does not affect the skin color beyond maximum whitening. After obtaining maximum whitening, a corresponding signal is provided to the user to quickly release the pressure. The measurement of the CFT is started at that moment, *t₀*. The skin coloring changes from maximum whitening and begins to return to essentially the original color. Normally, the rate of filling is higher at the beginning of the filling process, and is lower as time lapses. The apparatus uses the stored calibration value to determine the moment *t_{f}*, at which the skin color recovered and the measurement is stopped. The recovery time can be defined according to the desired measurement accuracy. For example, *t_{f}* can be defined as the moment at which the value of the digital word that corresponds to the current skin color reaches a value being 90% of the value of the digital word that corresponds to the original skin color of that specific patient. In the graph of Fig. 3A, the CFT is given by *t_{f}* - *t₀*.

According to a preferred embodiment of the invention, the accuracy of the measurement can also be determined by the rate of change in the skin coloring, in the time interval that is close to the end of the measurement. The last segment of the graph is represented between the time points *t₁* and *t_{f}*. The rate of change in this time interval is almost constant, and is almost not sensitive to the magnitude and duration of the applied pressure. Therefore, the CFT can be extrapolated with relative accuracy from the time interval *t_{f} - t₁*. The CFT under normal conditions should be below 1 Sec. A CFT value above 2 Sec can be suspected as a pre-shock state. Longer CFT is considered to indicate more severe shock state.

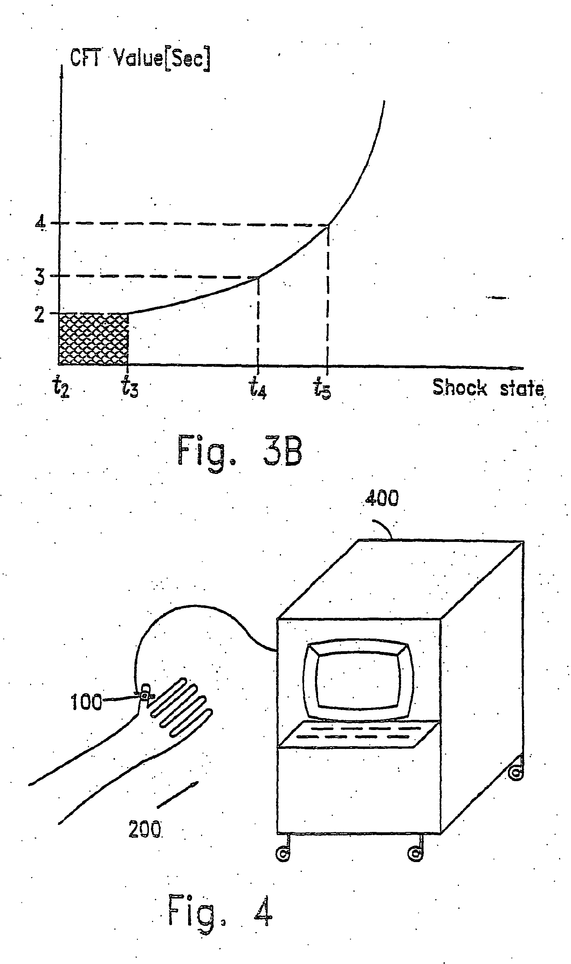

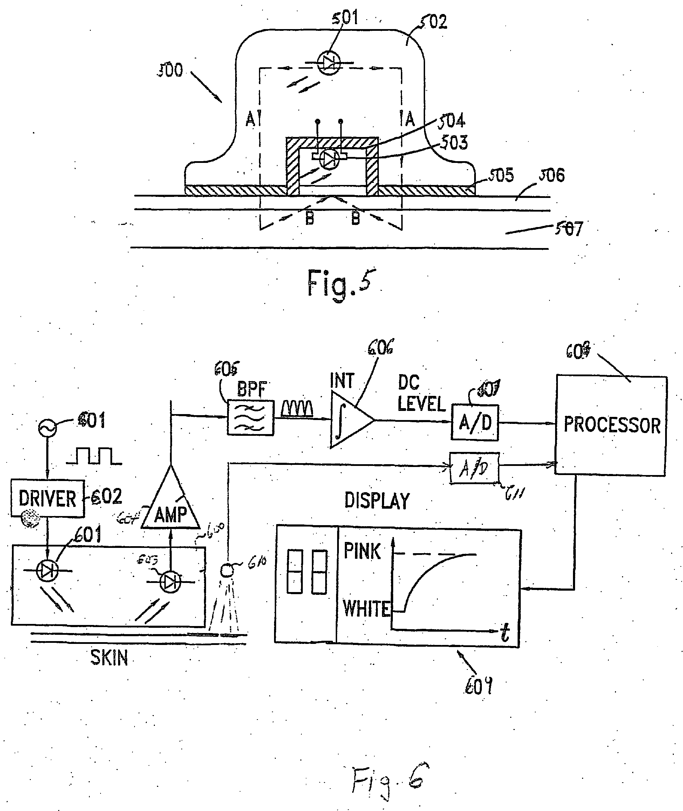

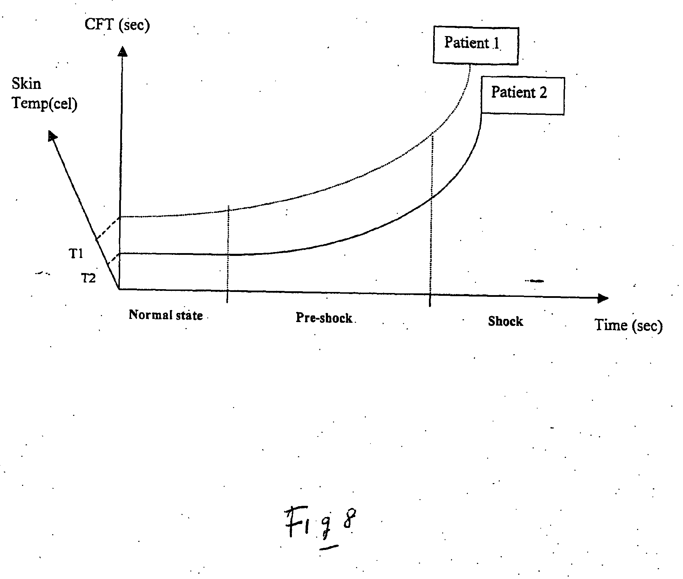

Fig. 3B is a graphical representation of the CFT as a function of the shock-state, for obtaining inferences related to the trend of the patient's physiological condition in reaction to medical treatment, according to a preferred embodiment of the invention. At the first time interval, between time-points *t₂* and *t₃*, the CFT value is below 2 seconds, and therefore, the patient is in normal condition (no shock). Early (mild) shock condition starts at time-points *t₃*, where the CFT value exceeds 2 seconds. As time lapses with no proper treatment, the shock becomes more severe, until the time-points *t₄*, which indicates the entry of the patient into a moderate shock condition(CFT value higher than 3 seconds). The next stage is indicated by the time-points *t₅*, which indicates the entry of the patient into a late (severe) shock condition (CFT value higher than 4 seconds). From point *t₅* and forth, the CFT grows rapidly.

According to a preferred embodiment of the invention, a medical treatment is given to the patient, and the CFT is measured from that moment on a periodic basis. This measurement is used to determine whether the pre-shock or the actual shock condition is reversible. If the patient's reaction to the given treatment is positive, as time lapses, the CFT is reduced, indicating a constant improvement in the physiological condition of the patient, until the CFT value is reduced below the 2 Sec margin.

Fig. 4 schematically illustrates the use an apparatus for the diagnosis of pre-shock state in a patient, according to a preferred embodiment of the invention. The apparatus 200 comprises a skin color sensing device 100 attached by straps or by adhesive materials to a skin area which is rich in subcutaneous blood vessels, such as hand fingers, and a processing and display unit 400. The sensing device 100 is connected to the processing and display unit 400 with electrical cord, through which the CFT data is fed for processing and display. Pressure is applied and released manually by the user, according to indications and/or instructions provided by the processing and display unit 400. The sensing device may also be attached to other locations in the patient's body that are rich in subcutaneous blood vessels, such as to the lip or to the ear lobe, for measuring the CFT.

According to a preferred embodiment of the invention, an automatic measurement can be carried out by integrating a controllable mechanical apparatus into the sensing device 100 and to control the applied pressure and the release of pressure by an external controller. Such mechanical apparatus may comprise a miniature linear motor that produces linear movement rather than rotational movement. Alternatively, linear movement pressure can be applied by an electromagnet, or by a rotational motor with an excentric movement mechanism. The linear movement can be controlled to depress a movable member, such as a movable transparent rigid layer, against the skin and to release the pressure by a corresponding control command.

According to a preferred embodiment of the invention, the sensing device 100 and the processing and display unit 400 may further comprise receiving and transmitting circuits to enable wireless exchange of data and control commands, required for CFT measurements. Wireless connection enables a single processing and display unit 400 to control and monitor several sensing devices 100, each of which is attached to a different patient. Each sensing devices 100 is identified by a unique code assigned to it, to eliminate wrong association between processed data and a patient.

The above examples and description have of course been provided only for the purpose of illustrations, and are not intended to limit the invention in any way. As will be appreciated by the skilled person, the invention can be carried out in a great variety of ways, employing more than one technique from those described above, such as using pneumatic apparatus for applying pressure on the patient skin, storing and displaying the history of CFT measurements of a patient, displaying the variation of the CFT curve with time, all without exceeding the scope of the invention.

## Claims

1. Apparatus for the diagnosis of physiological distress in a patient and of recovery of a patient from physiological distress, according to changes in the color of the patient's skin in response to an applied pressure on said skin, said pressure causing blood to flow out from blood vessels being subjacent to said skin, comprising:
a) image acquisition means (412) for acquiring an image of an area of the skin of said patient, to be gauged for color, said image acquisition means being positioned, so that said area is essentially within the focal plane of said image acquisition means, which image acquisition means comprises a color video camera (412) contained in a transparent external housing (102) so that most of the background light enters through the external housing and illuminates the skin surface (106);
b) circuitry (207) for obtaining a base-line color measurement using the acquired image data when essentially no pressure is applied;
c) circuitry for comparing the color of said area with the base-line color measurement, thereby determining the filling time of blood vessels in said area after releasing said pressure;

2. Apparatus according to claim 1, further comprising means for illuminating area of the skin to be gauged for color, with light radiation in a level being sufficient for enabling the image acquisition means to discriminate between colors.

3. Apparatus according to claim 1, wherein there is provided a transducer for applying pressure on said area, and for obtaining a signal value which corresponds to maximum whitening of said area.

4. Apparatus according to claim 1, further comprising circuitry for providing an alert signal whenever the strength and/or the duration of the applied pressure is not sufficient for obtaining maximum whitening.

5. Apparatus according to claim 1, further comprising a rigid transducer containing the image acquisition means and having a transparent wall being in contact with a limb of the patient, for applying a controlled pressure on the surface of said limb.

6. Apparatus according to claim 5, further comprising a linear motor integrated into the transducer, for applying automated and/or controlled force on said transducer.

7. Apparatus according to claim 5, further comprising an electromagnet integrated into the transducer, for applying automated and/or controlled force on said transducer.

8. Apparatus according to claim 5, further comprising a rotational motor having an eccentric movement mechanism to the transducer, for applying force on said transducer.

9. Apparatus according to claim 5, in which the transducer and/or the image acquisition means comprises circuitry for wireless data transmission and/or control data.

## Patentansprüche

1. Gerät zur Diagnose eines physiologischen Leidens eines Patienten und zur Erholung eines Patienten von einem physiologischen Leiden, gemäß Veränderungen der Hautfarbe des Patienten in Reaktion auf einen auf die Haut ausgeübten Druck, wobei der Druck bewirkt, dass Blut aus den unter der Haut liegenden Blutgefäßen wegströmt, umfassend:
a) ein Bilderfassungsmittel (412), um ein Bild eines Bereichs der Haut des Patienten zu erfassen, dessen Farbe gemessen werden soll, wobei das Bilderfassungsmittel so positioniert wird, dass sich der Bereich im Wesentlichen innerhalb der Fokalebene des Bilderfassungsmittels befindet, wobei das Bilderfassungsmittel eine Farbvideokamera (412) umfasst, die in einem transparenten äußeren Gehäuse (102) enthalten ist, sodass der Großteil des Hintergrundlichts durch das äußere Gehäuse eintritt und die Hautoberfläche (106) beleuchtet,
b) eine Schaltung (207), um eine Basis-Farbmessung unter Verwendung der erfassten Bilddaten zu erhalten, wenn im Wesentlichen kein Druck ausgeübt wird,
c) eine Schaltung zum Vergleichen der Farbe des Bereichs mit der Basis-Farbmessung, wodurch die Füllzeit von Blutgefäßen in dem Bereich bestimmt wird, nachdem der Druck entspannt wurde.

2. Gerät nach Anspruch 1, weiter umfassend Mittel zum Beleuchten des Bereichs der Haut, dessen Farbe gemessen werden soll, mit Lichtstrahlung auf einem Niveau, das ausreicht, um es dem Bilderfassungsmittel zu ermöglichen, zwischen Farben zu unterscheiden.

3. Gerät nach Anspruch 1, wobei ein Wandler bereitgestellt ist, um Druck auf den Bereich auszuüben und um einen Signalwert zu erhalten, der einem maximalen Blasswerden des Bereichs entspricht.

4. Gerät nach Anspruch 1, weiter umfassend eine Schaltung, um jedes Mal ein Alarmsignal bereitzustellen, wenn die Stärke und/oder die Dauer des ausgeübten Drucks nicht ausreicht, um ein maximales Weisswerden bzw. Blasswerden zu erreichen.

5. Gerät nach Anspruch 1, weiter umfassend einen starren Wandler, der das Bilderfassungsmittel enthält und der eine transparente Wand umfasst, die mit einer Extremität des Patienten in Kontakt steht, um einen gesteuerten Druck auf die Oberfläche der Extremität auszuüben.

6. Gerät nach Anspruch 5, weiter umfassend einen Linearmotor, der in dem Wandler integriert ist, um automatisch und/oder gesteuert Kraft auf den Wandler auszuüben.

7. Gerät nach Anspruch 5, weiter umfassend einen Elektromagnet, der in dem Wandler integriert ist, um automatisch und/oder gesteuert Kraft auf den Wandler auszuüben.

8. Gerät nach Anspruch 5, weiter umfassend einen Rotationsmotor mit einem Exzenter-Bewegungsmechanismus, um Kraft auf den Wandler auszuüben.

9. Gerät nach Anspruch 5, wobei der Wandler und/oder das Bilderfassungsmittel eine Schaltung zur drahtlosen Übertragung von Daten und/oder Steuerdaten umfasst.

## Revendications

1. Appareil de diagnostic de détresse physiologique chez un patient et de guérison d'un patient d'une détresse physiologique, en fonction de changements de la couleur de la peau du patient en réponse à une pression appliquée sur ladite peau, ladite pression amenant le sang à s'écouler hors des vaisseaux sanguins sous-jacents à ladite peau, comprenant :
a) un moyen d'acquisition d'image (412) pour acquérir une image d'une zone de la peau dudit patient, à calibrer pour la couleur, ledit moyen d'acquisition d'image étant positionné de sorte que ladite zone se trouve sensiblement à l'intérieur du plan focal dudit moyen d'acquisition d'image, ledit moyen d'acquisition d'image comprenant une caméra vidéo couleur (412) contenue dans un boîtier externe transparent (102) de sorte que la plupart de la lumière d'arrière-plan entre à travers le boîtier externe et illumine la surface de la peau (106) ;
b) un circuit (207) pour obtenir une mesure de couleur de référence en utilisant les données d'image acquise lorsque sensiblement aucune pression n'est appliquée ;
c) un circuit pour comparer la couleur de ladite zone à la mesure de couleur de référence, en déterminant de ce fait le temps de remplissage de vaisseaux sanguins dans ladite zone après avoir relâché ladite pression.

2. Appareil selon la revendication 1, comprenant en outre un moyen pour illuminer une zone de la peau à calibrer pour la couleur, avec un rayonnement de lumière à un niveau suffisant pour permettre au moyen d'acquisition d'image de différencier les couleurs.

3. Appareil selon la revendication 1, dans lequel il est prévu un transducteur pour appliquer une pression sur ladite zone et pour obtenir une valeur de signal qui correspond à une décoloration maximale de ladite zone.

4. Appareil selon la revendication 1, comprenant en outre un circuit pour fournir un signal d'alerte lorsque la force et/ou la durée de la pression appliquée n'est pas suffisante pour obtenir une décoloration maximale.

5. Appareil selon la revendication 1, comprenant en outre un transducteur rigide contenant le moyen d'acquisition d'image et ayant une paroi transparente en contact avec un membre du patient, pour appliquer une pression régulée sur la surface dudit membre.

6. Appareil selon la revendication 5, comprenant en outre un moteur linéaire intégré au transducteur pour appliquer une force automatisée et/ou régulée sur ledit transducteur.

7. Appareil selon la revendication 5, comprenant en outre un électroaimant intégré au transducteur pour appliquer une force automatisée et/ou régulée sur ledit transducteur.

8. Appareil selon la revendication 5, comprenant en outre un moteur rotationnel ayant un mécanisme de mouvement excentrique sur le transducteur pour appliquer une force sur ledit transducteur.

9. Appareil selon la revendication 5, dans lequel le transducteur et/ou le moyen d'acquisition d'image comprennent un circuit pour la transmission de données sans fil et/ou de données de commande.
